# EUROPEAN PATENT APPLICATION

(11) **EP 4 162 946 A1**
(43) Date of publication of application: **12.04.2023**
(21) Application number: 21306397.7
(22) Date of filing: 05.10.2021
(51) Int. Cl.: A61K 38/45, A61K 31/47

(54) **KYNURENINE AMINOTRANSFERASE AND PRODUCTS THEREOF FOR THE TREATMENT OF INFLAMMATORY BOWEL DISEASES**

(71) Applicant: Institut national de recherche pour l'agriculture, l'alimentation et l'environnement, 75007 Paris (FR); Institut des Sciences et Industries du Vivant et de l'Environnement, 75005 Paris (FR); SORBONNE UNIVERSITE, 75006 Paris (FR); Assistance Publique Hôpitaux de Paris, 75004 Paris (FR); Institut National de la Santé et de la Recherche Médicale (INSERM), 75013 Paris (FR)
(72) Inventor: SOKOL, Harry, 75015 PARIS (FR); MICHAUDEL, Chloé, 94260 FRESNES (FR); LANGELLA, Philippe, 78140 VELIZY (FR); AUCOUTURIER, Anne, 78140 VELIZY (FR); BERMUDEZ, Luis, 91400 SACLAY (FR)
(74) Representative: Cabinet Becker et Associés

(57) **Abstract**

The present invention relates to the treatment of inflammatory bowel diseases with a kynurenine aminotransferase, a living recombinant bacterium which has been genetically modified to express and secrete said kynurenine aminotransferase, and/or a product of said kynurenine aminotransferase which is xanthurenic acid, a derivative thereof, or any pharmaceutically acceptable salt or solvate thereof.

## Description

### FIELD OF THE INVENTION

The present invention relates to the field of medicine and in particular to compositions for the treatment of inflammatory bowel diseases.

### BACKGROUND OF THE INVENTION

Inflammatory bowel diseases (IBD) is a general term used to identify a group of inflammatory disorders of the gastrointestinal (GI) tract including Crohn's disease (CD) and ulcerative colitis (UC). While the exact cause of IBD is not entirely understood, it is known to involve genetic predisposition, dysbiosis of the gut microbiota and environmental influences. IBD is characterized by the repeated alternating cycles of clinical relapse and remission. In the absence of adequate treatment, IBD leads to chronic inflammation and thus to irreversible intestinal damages.

CD can affect any part of the GI tract but most commonly affects the end of the small intestine (the ileum) where it joins the beginning of the colon. CD may appear in "patches," affecting some areas of the GI tract while leaving other sections completely untouched. In CD, the inflammation may extend through the entire thickness of the bowel wall. UC is limited to the large intestine (colon) and the rectum. The inflammation occurs only in the innermost layer of the lining of the intestine. It usually begins in the rectum and lower colon, but may also spread continuously to involve the entire colon. In some individuals, it is difficult to determine whether their IBD is CD or UC. In these rare cases, people are given the diagnosis of indeterminate colitis (IC).

Some recent studies have revealed that aryl hydrocarbon receptor (AhR) agonists or bacterial probiotics producing such agonists may have a beneficial effect on IBD (see WO 2018/065132 and WO 2017/032739). However, despite increased understanding of the underlying disease process there is no cure. Medications currently available such as anti-inflammatory drugs corticosteroids and immune system suppressors, are used to reduce the inflammation that triggers signs and symptoms. However, the same treatments have significant potential toxicity including increased infection risk, increased risk of malignancy, and many patients can lose response when treatments are used long term.

Thus, there is still a strong need of new medications to be used in the treatment of IBD.

### SUMMARY OF THE INVENTION

The inventors herein demonstrated that administration of a kynurenine aminotransferase or one of its products, namely xanthurenic acid, provides an anti-inflammatory effect in mice submitted to DSS-induced colitis.

Accordingly, the present invention relates to a composition for use in the treatment of an inflammatory bowel disease, wherein said composition comprises
- a kynurenine aminotransferase (KAT),
- a living recombinant bacterium which has been genetically modified to express and secrete said kynurenine aminotransferase, and/or
- a product of said kynurenine aminotransferase which is xanthurenic acid, a derivative thereof, or any pharmaceutically acceptable salt or solvate thereof.

The inflammatory bowel disease is preferably selected from the group consisting of Crohn's disease, ulcerative colitis, indeterminate colitis (IC), other noninfective gastroenteritis, enteritis, enterocolitis and colitis, and pouchitis, more preferably selected from Crohn's disease and ulcerative colitis.

The kynurenine aminotransferase may be selected from the group consisting of human kynurenine--oxoglutarate transaminase 1 (KAT I), human kynurenine/alpha-aminoadipate aminotransferase (KAT II), human kynurenine-oxoglutarate transaminase 3 (KAT III), human mitochondrial aspartate aminotransferase (KAT IV), orthologs thereof, and variants thereof, said variants having at least 80% sequence identity to human KAT I, human KAT II, human KAT III, human KAT IV or to any ortholog thereof, and exhibiting kynurenine aminotransferase activity. In particular, the kynurenine aminotransferase may be selected from the group consisting of human KAT II, human KAT III, human KAT IV, orthologs thereof, and variants thereof, said variants having at least 80% sequence identity to human KAT II, human KAT III, human KAT IV or to any ortholog thereof, and exhibiting kynurenine aminotransferase activity. More particularly, the kynurenine aminotransferase may be selected from the group consisting of human KAT II, orthologs thereof, and variants thereof, said variants having at least 80% sequence identity to human KAT II or to any ortholog thereof, and exhibiting kynurenine aminotransferase activity.

In a particular embodiment, the kynurenine aminotransferase is selected from the group consisting of KAT proteins of SEQ ID NO: 1 to 32, and variants thereof having at least 80% sequence identity to any sequence of SEQ ID NO: 1 to 32 and exhibiting kynurenine aminotransferase activity. In a more particular embodiment, the kynurenine aminotransferase is selected from the group consisting of KAT proteins of SEQ ID NO: 10 to 16, and variants thereof having at least 80% sequence identity to any sequence of SEQ ID NO: 10 to 16 and exhibiting kynurenine aminotransferase activity.

The composition may comprise said kynurenine aminotransferase.

Alternatively or additionally, the composition may comprise a recombinant bacterium which has been genetically modified to express and secrete said kynurenine aminotransferase. Preferably, said recombinant bacterium is selected from the group consisting of bacteria belonging to the genera *Allobaculum, Adlercreutzia, Anaerostipes, Bifidobacterium, Propionibacterium, Bacteroides, Eubacterium, Enterococcus, Ruminococcus* and *Faecalibacterium, Escherichia coli,* and lactic acid bacteria such as bacteria belonging to the genera *Lactobacillus, Lactococcus* and *Streptococcus.*

Alternatively or additionally, the composition may comprise xanthurenic acid, a derivative thereof or any pharmaceutically acceptable salt or solvate thereof, wherein the xanthurenic acid derivative is of formula (I) 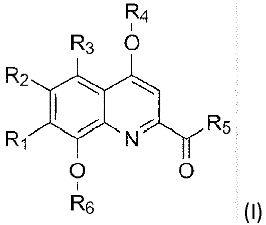 wherein
R₁, R₂ and R₃ are independently selected from the group consisting of a hydrogen atom, an oxygen atom, a halogen atom, a C₁₋₁₀ alkyl, C₂₋₁₀ alkenyl or C₂₋₁₀ alkynyl group optionally substituted by a halogen atom;
R₄ and R₆ are independently selected from the group consisting of a hydrogen atom and a C₁₋₁₀ alkyl group, or R₄ and/or R₆ are absent; and
R₅ is selected from the group consisting of a hydroxyl group, NHR₇, NR₇R₇ and a C₁₋₁₀ alkyl or alkoxy group, wherein R₇ is selected from the group consisting of a hydrogen atom and a C₁₋₁₀ alkyl group.

In particular, the xanthurenic acid derivative may be selected from the group consisting of oxo-xanthurenic acid (OXA) and di-oxo-xanthurenic acid (DOXA). Preferably, the composition comprises xanthurenic acid or any pharmaceutically acceptable salt or solvate thereof.

The composition may further comprise nicotinamide adenine dinucleotide or a precursor thereof, or may be to be used in combination with nicotinamide adenine dinucleotide or a precursor thereof.

### BRIEF DESCRIPTION OF THE FIGURES

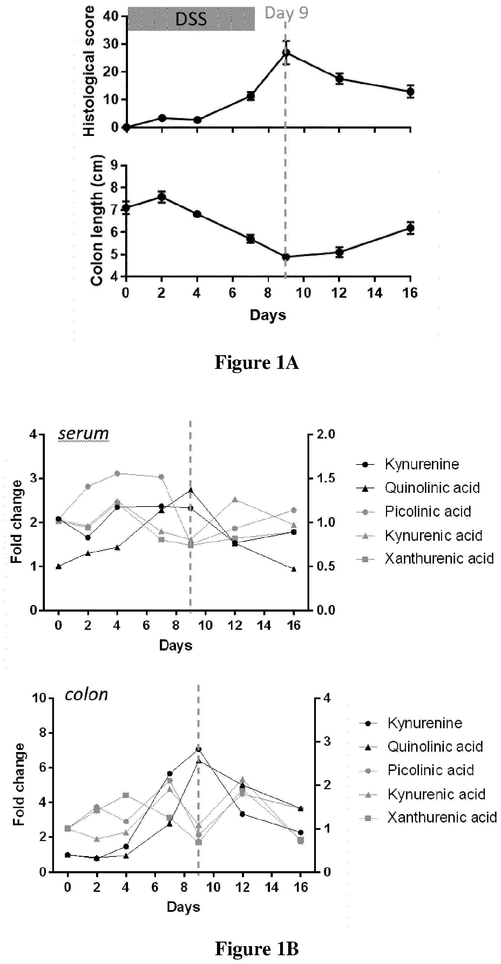

**Figure 1****:** kynurenic acid (KYNA) and xanthurenic acid (XANA) abundance are negatively correlated with inflammation during DSS-induced colitis. (**A**) histological scores and colon length dynamic during the course of the colitis n=8-10, (**B**) dynamic of kynurenine pathway metabolites in serum and colon tissue during the course of the colitis, n=8-10.

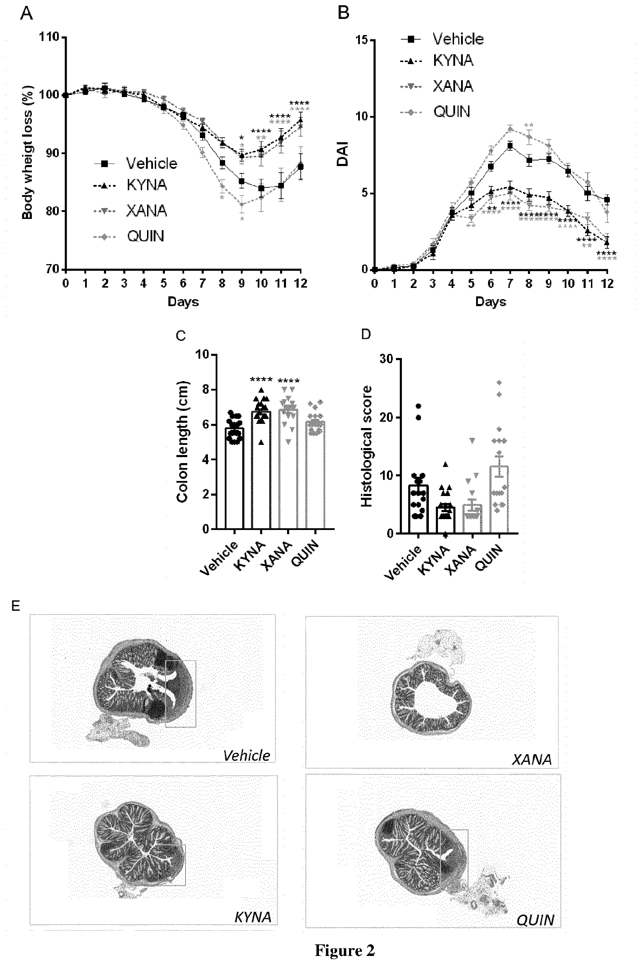

**Figure 2****:** KYNA and XANA protect against DSS-induced colitis, but not quinolinic acid (QUIN). Body weight loss (**A**), DAI (**B**), colon length (**C**), histological score (**D**), colon pictures (**E**). Statistical analysis: n=16-20 mice per group, Two-way or one-way ANOVA, with Bonferroni post test.

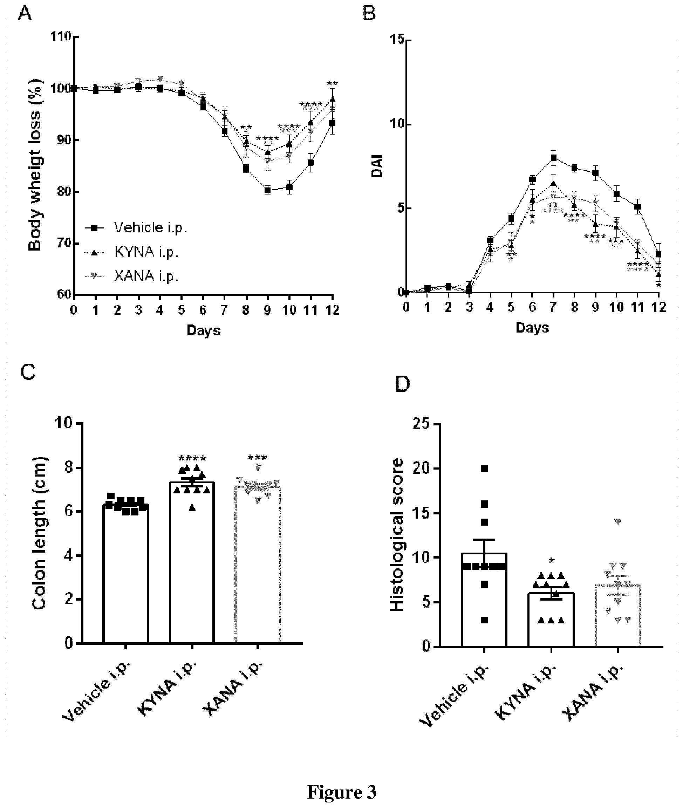

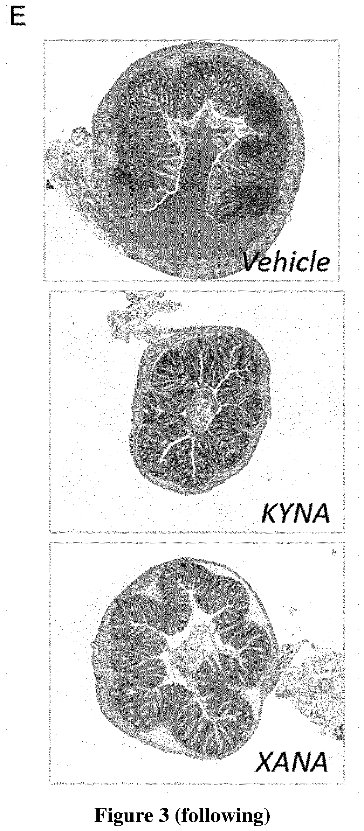

**Figure 3****:** Intraperitoneal injections of KYNA and XANA protect from colitis. Body weight loss (**A**), DAI (**B**), colon length (**C**), histological score (**D**), colon pictures (**E**). Statistical analysis : n=10 mice per group, Two-way or one-way ANOVA with Bonferroni post test.

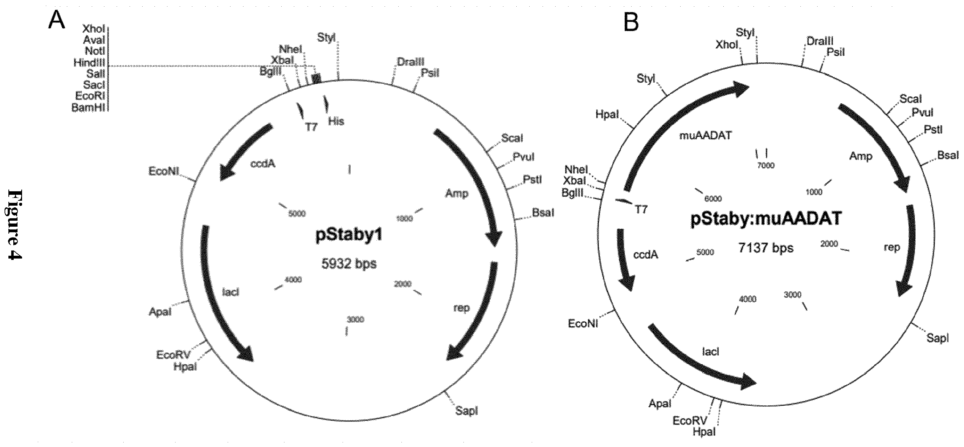

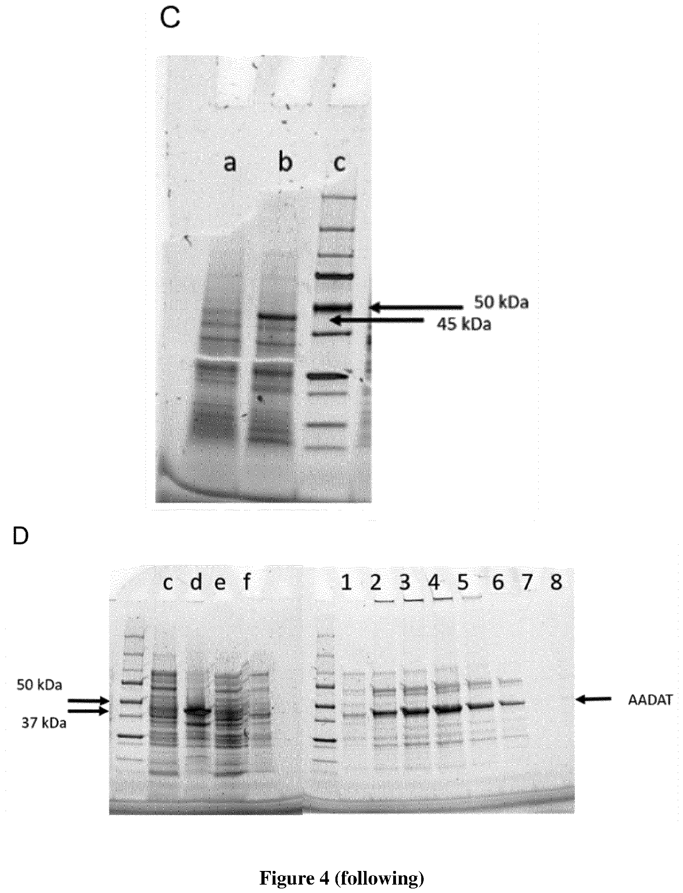

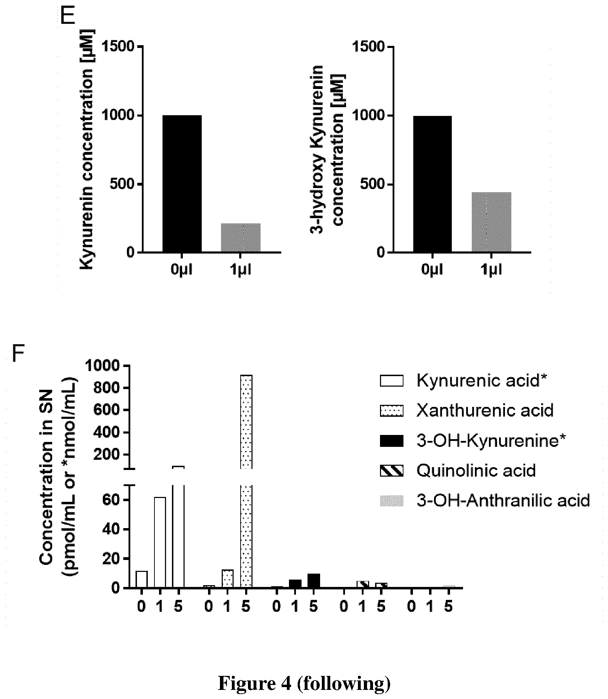

**Figure 4****:** AADAT production in *Escherichia coli.* The pStaby1 plasmid (**A**) and the muAADAT plasmid (**B**). Induction of the production of the AADAT protein. (**C**) The following samples were loaded on an acrylamide gel protein: before induction (a); after induction (b) and the unstained protein ladder Biorad (c). Visualisation of the produced and purified AADAT protein (**D**). The following samples were loaded on an acrylamide gel: culture supernatant (c); culture pellet (d); flow through (e), 40 mM imidazole wash (f) and elution fractions (1 to 8). Evaluation of AADAT activity through kynurenin and 3-hydroxy kynurenine degradation (**E**). Transformation of KYNU in KYNA, XANA and other metabolites with our AADAT production (**F**).

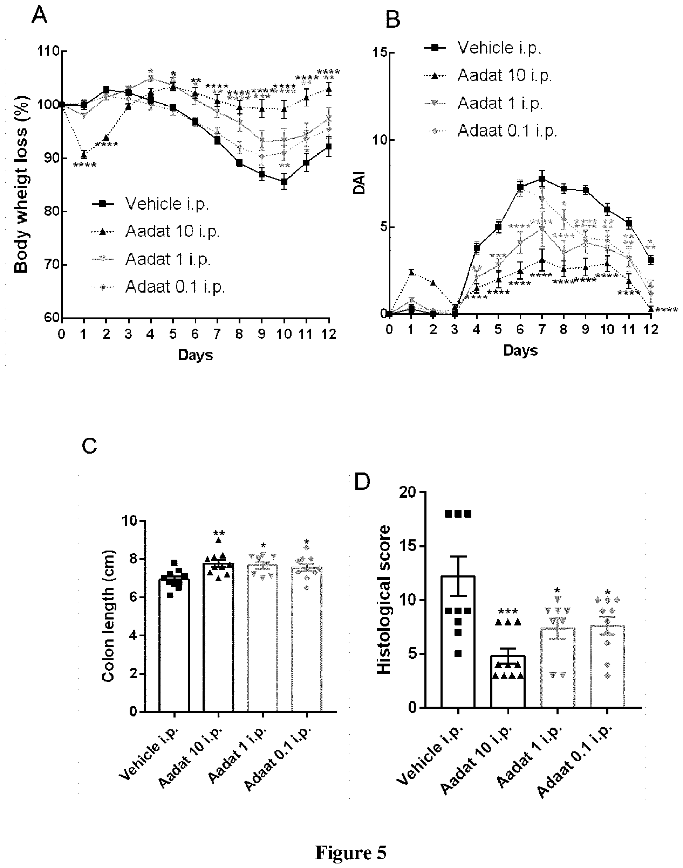

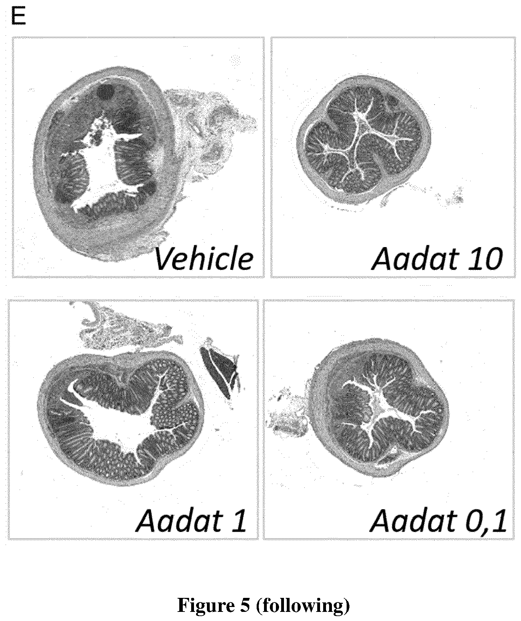

**Figure 5****:** AADAT injections protect during DSS-induced colitis. Body weight loss (**A**), DAI (**B**), colon length (**C**), histological scores (**D**) and colon pictures (**E**). Statistical analysis: n=8-10 mice per group, Two-way or one-way ANOVA with Bonferroni post test.

### DETAILED DESCRIPTION OF THE INVENTION

The inventors herein demonstrated in a mouse model of intestinal inflammation induced by DSS that xanthurenic acid (XANA) and kynurenic acid (KYNA) negatively correlate with inflammation while kynurenine and quinolinic acid have the opposite dynamic. They further confirmed this result in a human context wherein a negative correlation was observed between several markers of Crohn's disease activity with KYNA and XANA. Based on these results, they also showed that oral or intraperitoneal administration of XANA and KYNA in mice submitted to DSS-induced colitis led to a lower weight loss, a lower activity index of colitis, a longer colon, a lower histological score and a reduced expression of pro-inflammatory cytokines thereby demonstrating the systemic anti-inflammatory effect of XANA and KYNA. Similar results were obtained by intraperitoneal administration of a purified kynurenine aminotransferase, in particular an alpha-aminoadipate aminotransferase (AADAT or KAT II) in a mouse model of colitis induced by DSS. The results showed protection against inflammation in terms of weight loss and clinical score, but also colon length or histological score, even at the lowest dose. The inventors therefore herein demonstrated that administration of kynurenine aminotransferase or xanthurenic acid shows a strong therapeutic potential to treat or prevent inflammatory bowel diseases.

Accordingly, in a first aspect; the present invention relates to a composition for use in the treatment of an inflammatory bowel disease.

Said composition may comprise
- a kynurenine aminotransferase,
- a live recombinant bacterium which has been genetically modified to express and secrete said kynurenine aminotransferase, and/or
- xanthurenic acid or a derivative thereof, or any pharmaceutically acceptable salt thereof.

As used herein, the term "kynurenine aminotransferase" or "KAT" refers to an enzyme that catalyzes the transamination of kynurenine to form kynurenic acid and/or the transamination of 3-hydroxykynurenine to form xanthurenic acid.

As used herein, "KAT activity" refers to the catalysis of the transamination of kynurenine to form kynurenic acid and/or the transamination of 3-hydroxykynurenine to form xanthurenic acid, preferably the catalysis of 3-hydroxykynurenine to form xanthurenic acid. KAT activity may be assessed by any method known by the skilled person. For instance, KAT activity could be assessed as described in the experimental section, i.e. an assay based on the disappearance of kynurenine and/or 3-hydroxykynurenin. Briefly, a reaction mixture (50 µL final volume) containing 10 mM L-kynurenine or 3-hydroxykynurenin, 2 mM α-oxoglutarate, 40 µM PLP (pyridoxal 5'-phosphate) and 0 or 1 µL of the protein to be tested prepared in buffer 100 mM potassium phosphate (pH 7.4), is incubated at 37°C for 15 min. The reaction is stopped by the addition of an equal volume of 30% acetic acid. The supernatant of the reaction mixture, obtained by centrifugation at 3000 g for 10 min at 4 °C, is mixed equally with Ehrlich's solution and incubated 15 min at room temperature to have a colorimetric reaction. In parallel, a standard range of kynurenine or 3-hydroxykynurenin from 0 µM to 1000 µM is made under the same conditions. The amount of kynurenine or 3-hydroxykynurenin present in the sample is measured with a spectrophotometer at an OD of 492 nm and calculated using the standard range. The disappearance of kynurenine and/or 3-hydroxykynurenin indicates that the protein exhibits KAT activity. More particularly, the disappearance of kynurenine indicates that the protein is able to catalyze the transamination of kynurenine to form kynurenic acid and the disappearance of 3-hydroxykynurenine indicates that the protein is able to catalyze the transamination of 3-hydroxykynurenine to form xanthurenic acid. The production of kynurenic acid and/or xanthurenic acid can be confirmed by mass spectrometry.

Four KAT have been reported in mammals: kynurenine-oxoglutarate transaminase 1 (KAT I), kynurenine/alpha-aminoadipate aminotransferase (KAT II or AADAT), kynurenine-oxoglutarate transaminase 3 (KAT III) and mitochondrial aspartate aminotransferase (KAT IV). In the present invention, the kynurenine aminotransferase may be a human KAT or an orthologous protein. The term "ortholog" or "orthologous protein" as used herein refers a functional counterpart (i.e. exhibiting KAT activity) of a protein in another species. Orthologous proteins are similar to each other because they originated from a common ancestor. Sequence differences between the orthologs are thus the result of speciation. The orthologous sequences can be encompassed in longer or shorter isoforms. Methods for identification of orthologous proteins are well known in the art.

Human kynurenine-oxoglutarate transaminase 1 (KAT I) is encoded by the gene *KYAT1,* also named *CCBL1* (Uniprot accession number: Q16773). Three isoforms of human KAT I are produced by alternative splicing: isoform 1 (SEQ ID NO:1), isoform 2 (SEQ ID NO: 2) and isoform 3 (SEQ ID NO:3).

Orthologous proteins of human KAT I may also be used in the present invention. Preferably, orthologous proteins of human KAT I used in the present invention are mammalian proteins. Examples of orthologous proteins of human KAT I that may be used in the present invention include, but are not limited to, the orthologs listed in Table 1.

**Table 1: examples of orthologs of human KAT I**

| **Organism** | **Uniprot accession number** | **Sequences** |
|---|---|---|
| Rattus norvegicus (Rat) | Q08415 | SEQ ID NO: 4 (isoform 1) |
| | | SEQ ID NO: 5 (isoform 2) |
| Mus musculus (Mouse) | Q8BTY1 | SEQ ID NO:6 |
| Pan troglodytes (Chimpanzee) | G2HET0 | SEQ ID NO:7 |
| Macaca mulatta (Rhesus macaque) | F7EI86 | SEQ ID NO:8 |
| Bos taurus (Bovine) | A0JN49 | SEQ ID NO:9 |

Human kynurenine/alpha-aminoadipate aminotransferase (KAT II or AADAT) is encoded by the gene *AADAT* also named *KYAT2* or *KAT2* (Uniprot accession number: Q8N5Z0). Two isoforms of human KAT II are produced by alternative splicing: isoform 1 (SEQ ID NO: 10) and isoform 2 (SEQ ID NO:11).

Orthologous proteins of human KAT II may also be used in the present invention. Preferably, orthologous proteins of human KAT II used in the present invention are mammalian proteins. Examples of orthologous proteins of human KAT II that may be used in the present invention include, but are not limited to, the orthologs listed in Table 2.

**Table 2: examples of orthologs of human KAT II**

| **Organism** | **Uniprot accession number** | **Sequences** |
|---|---|---|
| Rattus norvegicus (Rat) | Q64602 | SEQ ID NO: 12 |
| Mus musculus (Mouse) | Q9WVM8 | SEQ ID NO:13 |
| Pan troglodytes (Chimpanzee) | H2QQF3 | SEQ ID NO:14 |
| Macaca mulatta (Rhesus macaque) | H9EWA2 | SEQ ID NO:15 |
| Bos taurus (Bovine) | Q5E9N4 | SEQ ID NO:16 |

Human kynurenine--oxoglutarate transaminase 3 (KAT III or CCBL2) is encoded by the gene *KYAT3* (Uniprot accession number: Q6YP21). Three isoforms of human KAT III are produced by alternative splicing: isoform 1 (SEQ ID NO: 17), isoform 2 (SEQ ID NO:18) and isoform 3 (SEQ ID NO:19).

Orthologous proteins of human KAT III may also be used in the present invention. Preferably, orthologous proteins of human KAT III used in the present invention are mammalian proteins. Examples of orthologous proteins of human KAT III that may be used in the present invention include, but are not limited to, the orthologs listed in Table 3.

**Table 3: examples of orthologs of human KAT III**

| **Organism** | **Uniprot accession number** | **Sequences** |
|---|---|---|
| Rattus norvegicus (Rat) | Q58FK9 | SEQ ID NO: 20 |
| Mus musculus (Mouse) | Q71RI9 | SEQ ID NO:21 (isoform 1) |
| | | SEQ ID NO:22 (isoform 2) |
| Pan troglodytes (Chimpanzee) | H2PZC7 | SEQ ID NO:23 |
| Macaca mulatta (Rhesus macaque) | H9FX77 | SEQ ID NO:24 |
| Bos taurus (Bovine) | Q0P5G4 | SEQ ID NO:25 |

Human mitochondrial aspartate aminotransferase (KAT IV) is encoded by the gene *GOT2,* also named *KYAT4* (Uniprot accession number: P00505). Two isoforms of human KAT IV are produced by alternative splicing: isoform 1 (SEQ ID NO: 26) and isoform 2 (SEQ ID NO:27).

Orthologous proteins of human KAT IV may also be used in the present invention. Preferably, orthologous proteins of human KAT IV used in the present invention are mammalian proteins. Examples of orthologous proteins of human KAT IV that may be used in the present invention include, but are not limited to, the orthologs listed in Table 4.

**Table 4: examples of orthologs of human KAT IV**

| **Organism** | **Uniprot accession number** | **Sequences** |
|---|---|---|
| Rattus norvegicus (Rat) | P00507 | SEQ ID NO: 28 |
| Mus musculus (Mouse) | P05202 | SEQ ID NO:29 |
| Pan troglodytes (Chimpanzee) | H2QB89 | SEQ ID NO:30 |
| Macaca mulatta (Rhesus macaque) | F6UDV1 | SEQ ID NO:31 |
| Bos taurus (Bovine) | P12344 | SEQ ID NO:32 |

In an embodiment, the kynurenine aminotransferase comprised in the composition or expressed by the recombinant bacterium may be selected from the group consisting of
- human KAT I, human KAT II, human KAT III, human KAT IV, and orthologs thereof, and
- variants having at least 80% sequence identity to human KAT I, human KAT II, human KAT III, human KAT IV or to any ortholog thereof, and exhibiting KAT activity.

Preferably, the kynurenine aminotransferase comprised in the composition or expressed by the recombinant bacterium is selected from the group consisting of KAT proteins of SEQ ID NO: 1 to 32, and variants thereof having at least 80% sequence identity to any sequence of SEQ ID NO: 1 to 32 and exhibiting KAT activity.

The term "variant", as used herein, refers to an enzyme which is derived from a wild-type kynurenine aminotransferase, a human kynurenine aminotransferase or an ortholog thereof, and comprises an alteration, i.e., a substitution, insertion, and/or deletion, at one or more (e.g., several) positions. The term "deletion", used in relation to a position or an amino acid, means that the amino acid in the particular position has been deleted or is absent. The term "insertion", used in relation to a position or amino acid, means that one or more amino acids have been inserted or are present adjacent to and immediately following the amino acid occupying the particular position. The variant may be obtained by various techniques well known in the art. In particular, examples of techniques for altering the DNA sequence encoding the wild-type protein, include, but are not limited to, site-directed mutagenesis, random mutagenesis and synthetic oligonucleotide construction.

As used herein, the term "sequence identity" or "identity" refers to the number (%) of matches (identical amino acid residues) in positions from an alignment of two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. Preferably, for purposes herein, % amino acid sequence identity values refers to values generated using a local alignment algorithm, preferably the Basic Local Alignment Search Tool (BLAST) that finds regions of local similarity between sequences and calculates the statistical significance of matches, wherein all search parameters are set to default values, i.e. blastp algorithm, Expect threshold= 0.05, word size= 3, Scoring matrix = BLOSUM62, Gap costs: existence=11, extension = 1, Conditional compositional score matrix adjustment.

In another embodiment, the kynurenine aminotransferase comprised in the composition or expressed by the recombinant bacterium is selected from the group consisting
- human KAT II, human KAT III, human KAT IV, and orthologs thereof, and
- variants having at least 80% sequence identity to human KAT II, human KAT III, human KAT IV or to any ortholog thereof and exhibiting KAT activity.

Preferably, the kynurenine aminotransferase comprised in the composition or expressed by the recombinant bacterium is selected from the group consisting of KAT proteins of SEQ ID NO: 10 to 32, and variants thereof having at least 80% sequence identity to any sequence of SEQ ID NO: 10 to 32 and exhibiting KAT activity.

In a preferred embodiment, the kynurenine aminotransferase comprised in the composition or expressed by the recombinant bacterium is selected from the group consisting
- human KAT II and orthologs thereof, and
- variants having at least 80% sequence identity to human KAT II or to any ortholog thereof and exhibiting KAT activity.

Preferably, the kynurenine aminotransferase comprised in the composition or expressed by the recombinant bacterium is selected from the group consisting of KAT proteins of SEQ ID NO: 10 to 16, and variants thereof having at least 80% sequence identity to any sequence of SEQ ID NO: 10 to 16 and exhibiting KAT activity.

In some embodiments, the kynurenine aminotransferase comprised in the composition or expressed by the recombinant bacterium may be fused at its N-terminus and/or C-terminus to another polypeptide to create a hybrid polypeptide or fusion polypeptide. Techniques for producing fusion polypeptides are known in the art, and include ligating the coding sequences encoding the variant and the addition region of another polypeptide so that they are in frame and that expression of the fusion polypeptide is under control of the same promoter(s) and terminator. The addition region of the fusion polypeptide can be selected in order to enhance the stability of the enzyme, to promote the secretion (such as a N-terminal hydrophobic signal peptide) of the fusion protein from a cell (such as a bacterial cell), or to assist in the purification of the fusion protein. More particularly, the additional region can be a tag useful for purification or immobilization of the enzyme. Such a tag is well-known by the person skilled in the art, for instance a His tag (His₆), a FLAG tag, a HA tag (epitope derived from the Influenza protein haemagglutinin), a maltose-binding protein (MPB), a MYC tag (epitope derived from the human proto-oncoprotein MYC) or a GST tag (small glutathione-S-transferase). A fusion polypeptide can further comprise a cleavage site for proteases or chemical agents, between the enzyme and the addition region. Upon secretion of the fusion protein, the site is cleaved releasing the two separate polypeptides. The kynurenine aminotransferase may also be fused at its N-terminus and/or C-terminus to one or several polypeptides exhibiting distinct enzymatic activity. Optionally, it may be also modified (e.g., chemically, enzymatically, physically, etc.) to improve one of its features such as stability or activity.

In some embodiments, the composition used in the present invention comprises a kynurenine aminotransferase, i.e. a protein exhibiting KAT activity and as defined above.

The kynurenine aminotransferase may be produced by known method such as recombinant techniques. In particular, it may be expressed and secreted from a host cell, preferably a recombinant bacterium as defined below or as illustrated in the examples, and isolated or purified. As used herein, the term "host cell" means any cell type that is susceptible to transformation, transfection, transduction, or the like with a nucleic acid construct or expression vector comprising a polynucleotide encoding a kynurenine aminotransferase as used in the present invention, and that is able to express said enzyme.

In particular, the kynurenine aminotransferase may be produced by a method comprising (a) culturing a host cell, in particular a recombinant bacterium as defined below, in a suitable culture medium under suitable conditions to express the kynurenine aminotransferase; and (b) recovering said kynurenine aminotransferase from the cell culture.

The host cells are cultivated in a nutrient medium suitable for production of polypeptides using methods known in the art. For example, the cell may be cultivated by shake flask cultivation, or small-scale or large-scale fermentation (including continuous, batch, fedbatch, or solid state fermentations) in laboratory or industrial fermentors performed in a suitable medium and under conditions allowing the enzyme to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection).

The enzyme may be recovered using any method known in the art. For example, the enzyme may be recovered from the nutrient medium by conventional procedures including, but not limited to, collection, centrifugation, filtration, extraction, spray-drying, evaporation, or precipitation. Optionally, the enzyme may be partially or totally purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction to obtain substantially pure polypeptides. Preferably, the enzyme is secreted into the nutrient medium and can be recovered directly from the culture supernatant before to be isolated or purified.

In embodiments wherein the composition comprises a kynurenine aminotransferase, said kynurenine aminotransferase is preferably in isolated or purified form.

As used herein, the term "isolated", in relation to a polypeptide, refers to a polypeptide that have been separated from at least one associated substance found in a source material (e.g., separated from at least one other cellular component) or any material associated with the protein in any process used to produce the preparation. For instance, an isolated polypeptide is typically devoid of at least some proteins or other constituents of the cells, e.g. recombinant cells, to which it is normally associated or with which it is normally admixed or in solution.

As used herein, the term "purified" means that the protein is essentially free of association with other proteins, as is for example the product purified from the culture of recombinant host cells or the product purified from a non-recombinant source. The term "purified" does not require absolute purity, rather it is intended as a relative definition. It denotes a protein that is essentially free from other components as determined by analytical techniques well known in the art (e.g., a purified protein forms a discrete band in an electrophoretic gel, chromatographic eluate, and/or a media subjected to density gradient centrifugation). A purified protein is at least 50 percent pure, usually at least 75, 80, 85, 90, 95, 96, 97, 98, 99 percent pure (e.g., percent by weight on a molar basis).

The kynurenine aminotransferase may be administered in a mature form or in the form of a precursor.

KAT used in the present invention, and in particular contained in the composition, may be modified in various ways. For example, one or more amino acids of L configuration may be replaced with amino acids of D configuration. The polypeptide may undergo a post-translational modification and/or an additional chemical modification, in particular a glycosylation, an amidation, an acylation, an acetylation or a methylation. Protective groups may also be added to the C-terminal and/or N-terminal ends. For example, the protective group at the N-terminal end may be an acylation or an acetylation and the protective group at the C-terminal end may be an amidation or an esterification. The polypeptide may also comprise pseudopeptide bonds replacing the "conventional" CONH peptide bonds and conferring increased resistance to peptidases, such as CHOH-CH₂, NHCO, CH₂-O, CH₂CH₂, CO-CH₂, N-N, CH=CH, CH₂NH, and CH₂-S. One or more amino acids may also be replaced with rare amino acids, in particular hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine and aminobutyric acid; or synthetic amino acids, in particular ornithine, norleucine, norvaline and cyclohexylalanine.

The invention also covers the use of the pharmaceutically acceptable salts of such polypeptides. The pharmaceutically acceptable salts may be, for example, the salts with pharmaceutically acceptable inorganic acids, such as hydrochloric acid, hydrobromic acid, sulphuric acid and phosphoric acid; the salts with pharmaceutically acceptable organic acids, such as acetic acid, citric acid, maleic acid, malic acid, succinic acid, ascorbic acid and tartaric acid; the salts with pharmaceutically acceptable inorganic bases, such as the sodium, potassium, calcium, magnesium or ammonium salts; or the salts with organic bases which have a salifiable nitrogen, commonly used in the pharmaceutical art. The methods for preparing these salts are well known to those skilled in the art.

The composition used in the present invention may comprise one or several KAT enzymes, i.e. may comprise one or several KAT enzymes
(i) selected from the group consisting of
   - human KAT I, human KAT II, human KAT III, human KAT IV, and orthologs thereof, and
   - variants having at least 80% sequence identity to human KAT I, human KAT II, human KAT III, human KAT IV or to any ortholog thereof, and exhibiting KAT activity; or
(ii) selected from the group consisting of
   - human KAT II, human KAT III, human KAT IV, and orthologs thereof, and
   - variants having at least 80% sequence identity to human KAT II, human KAT III, human KAT IV or to any ortholog thereof and exhibiting KAT activity, or
(iii) selected from the group consisting of
   - human KAT II and orthologs thereof, and
   - variants having at least 80% sequence identity to human KAT II or to any ortholog thereof and exhibiting KAT activity.

Preferably, the pharmaceutical composition used in the present invention comprises one or several KAT enzymes selected from the group consisting of
- human KAT II and orthologs thereof, and
- variants having at least 80% sequence identity to human KAT II or to any ortholog thereof and exhibiting KAT activity.

In some embodiments, the composition used in the present invention comprises a recombinant bacterium which has been genetically modified to express and secrete a kynurenine aminotransferase, i.e. a protein exhibiting KAT activity and as defined above.

As used herein, the term "recombinant bacterium" or "genetically modified bacterium" refers to a bacterium that is not found in nature and which contains a modified genome as a result of either a deletion, insertion or modification of its genetic elements. In particular, this term refers to a bacterium comprising a heterologous nucleic acid, expression cassette or vector as described below, i.e. a nucleic acid, cassette or vector that does not naturally occur in said bacterium.

As used herein, the term "endogenous", with respect to a bacterium, refers to a genetic element or a protein naturally present in said bacterium. The term "heterologous", with respect to a bacterium, refers to a genetic element or a protein that is not naturally present in said bacterium.

The recombinant bacterium used in the present invention has been genetically modified by introducing a heterologous expression cassette or vector comprising a nucleic acid encoding a KAT enzyme as defined above.

The nucleic acid encoding a KAT enzyme may be deduced from the sequence of the protein and codon usage may be adapted according to the bacterium in which the nucleic acid shall be transcribed. These steps may be carried out according to methods well known to one of skill in the art and some of which are described in the reference manual Sambrook *et al.* (Sambrook J, Russell D (2001) Molecular cloning: a laboratory manual, Third Edition Cold Spring Harbor).

An expression cassette as used herein comprises a nucleic acid encoding a KAT enzyme as defined above, operably linked to one or more control sequences that direct the expression of said nucleic acid in the recombinant bacterium under conditions compatible with the control sequences.

The control sequence may include a promoter that is recognized by the recombinant bacterium. The promoter contains transcriptional control sequences that mediate the expression of the KAT enzyme. The promoter may be any polynucleotide that shows transcriptional activity in the recombinant bacterium including mutant, truncated, and hybrid promoters and may be obtained from genes encoding extracellular or intracellular polypeptides either homologous or heterologous to the bacterium. The promoter may be a strong, weak, constitutive or inducible promoter. Usually, the promoter is heterologous to the nucleic acid encoding a KAT enzyme, i.e. is not operably linked in nature to said nucleic acid or is operably linked at a different location in nature. The promoter should be chosen in order to show transcriptional activity in the recombinant bacterium.

Examples of suitable promoters include, but are not limited to, constitutive promoters such as P21, P23, P32, P44 or P59 (Morello et al., J Mol Microbiol Biotechnol 2008;14:48-58) or inducible promoters such as nisin-inducible controlled gene expression (NICE) system derived from the nis (nisABTCIPRKEFG) operon present in some L. lactis strains (Kuipers et al., J Biotechnol 1998;64:15-21).

The control sequence may also be a transcription terminator, which is recognized by the bacterium to terminate transcription. The terminator is operably linked to the 3'-terminus of the nucleic acid encoding the KAT enzyme. Any terminator that is functional in the bacterium may be used in the present invention. Usually, the terminator is chosen in correlation with the promoter. Examples of suitable terminators include, but are not limited to, the rho-independent transcription terminator trpA (Christie et al. 1981, Proc. Natl. Acad. Sci. USA 78:4180-4184.), the termination region of the *rrnB* gene coding for ribosomal RNA in Escherichia coli (Orosz et al. Eur J Biochem. 1991 Nov 1;201(3):653-9).

The control sequence may also be a signal peptide coding sequence that encodes a signal peptide linked to the N-terminus of the encoded polypeptide and directs the polypeptide into the cell's secretory pathway, i.e. for secretion into the extracellular (or periplasmic) space. Any signal peptide coding sequence that directs the expressed polypeptide into the secretory pathway of the bacterium may be used. The signal peptide coding sequence can be cleaved by a number of signal peptidases, thus removing it from the rest of the expressed polypeptide. Examples of effective signal peptide coding sequences for bacterial host cells include, but are not limited to the signal peptide of Usp45 (SPUsp45), the major Sec-dependent protein secreted by *Lactococcus lactis* (Borrero et al., 2011. Applied Microbiology and Biotechnology 89(1):131-43), signal peptide SP310 (Ravn et al. Microbiology (Reading). 2003 Aug;149(Pt 8):2193-2201), and signal peptide Exp4 (US patent application US2006199246). It may also be desirable to add regulatory sequences that regulate expression of the KAT enzyme relative to the growth of the recombinant bacterium. Examples of regulatory systems are those that cause expression of the gene to be turned on or off in response to a chemical or physical stimulus, including the presence of a regulatory compound. Regulatory systems in prokaryotic systems include the lac, tac, and trp operator systems.

Typically, the expression cassette comprises, or consists of, a nucleic acid encoding a KAT enzyme operably linked to a transcriptional promoter and a transcription terminator. The expression cassette may also comprises several nucleic acids encoding several KAT enzymes operably linked to a transcriptional promoter and a transcription terminator. Preferably, the expression cassette further comprises a signal peptide coding sequence leading to secretion of the KAT enzyme(s) into the extracellular space.

The expression cassette may be used directly to transform the bacterium or may be introduced into an expression vector and said vector may be used to transform the bacterium.

The choice of the vector will typically depend on the compatibility of the vector with the bacterium into which the vector is to be introduced. The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the bacterium, is integrated into the genome and replicated together with the chromosome(s) into which it has been integrated.

The vector preferably comprises one or more selectable markers that permit easy selection of bacteria comprising the vector. A selectable marker is a gene the product of which provides for biocide or viral resistance, resistance to heavy metals, prototrophy to auxotrophy, and the like. Examples of bacterial selectable markers include, but are not limited to, markers that confer antibiotic resistance such as ampicillin, chloramphenicol, kanamycin, neomycin, spectinomycin, or tetracycline resistance.

The vector preferably comprises an element that permits integration of the vector into the bacterium's genome or autonomous replication of the vector in the cell independent of the genome. When integration into the host cell genome occurs, integration of the sequences into the genome may rely on homologous or non-homologous recombination. In one hand, the vector may contain additional polynucleotides for directing integration by homologous recombination at a precise location into the genome of the host cell. These additional polynucleotides may be any sequence that is homologous with the target sequence in the genome of the host cell. On the other hand, the vector may be integrated into the genome of the host cell by non-homologous recombination.

For autonomous replication, the vector may further comprise an origin of replication enabling the vector to replicate autonomously in the bacterium in question. The origin of replication may be any plasmid replicator mediating autonomous replication that functions in a cell. The term "origin of replication" or "plasmid replicator" means a polynucleotide that enables a plasmid or vector to replicate *in vivo.* Examples of bacterial origins of replication include, but are not limited to, the origins of replication of plasmids pBR322, pUC19, pACYC177, and pACYC184 permitting replication in *E. coli,* and pUB1 10, pE194, pTA1060, and pAMβ1 permitting replication in Bacillus.

The methods for selecting these elements according to the bacterium in which expression is desired, are well known to the skilled person. The vectors may be constructed by the classical techniques of molecular biology, well known to one of skill in the art.

The recombinant bacterium used in the present invention can be obtained by introducing an expression cassette or vector as described above so that the cassette or vector is maintained as a chromosomal integrant or as a self-replicating extra-chromosomal vector. Introduction of said expression cassette or vector into the bacterium may be carried out using any method well-known by the skilled person. Preferably, at least the expression cassette, or a part thereof comprising a nucleic acid encoding KAT enzyme(s) and enabling the expression of said nucleic acid, is integrated into the genome of the bacterium.

The recombinant bacterium contained in the composition used in the invention is a living bacteria. This bacterium may be a Live Biotherapeutic Product (LBP), i.e. a live organism designed and developed to treat, cure, or prevent a disease or condition in a subject. By "living bacteria", it is to be understood that the integrity of the cells is maintained and that cellular processes occur or can occur if the bacteria are cultured in the suitable medium and conditions. A living bacterium can be re-seeded in a suitable culture medium and grow under suitable conditions. Living bacteria may be preserved before administration by freezing with liquid nitrogen, gradual freezing or lyophilization and subsequent storage, preferably at temperatures ranging from +4 °C to -80 °C.

The recombinant bacterium may be any bacterium that can be administered to a subject without inducing disease (nonpathogenic bacterium) and having a metabolism adapted to the gastrointestinal tract environment. The recombinant bacterium may also have been genetically engineered to improve its pharmacokinetic and pharmacodynamic properties. For example, the bacterium may have been modified to exhibit auxotrophy in order to limit bacterial replication in the absence of a provided metabolite. Examples and strategies to develop chassis organisms for engineered live bacterial therapeutics have been recently reviewed (Charbonneau et al. 2020, Nature Communications volume 11, Article number: 1738) and the skilled person may easily chose a bacterial chassis adapted to the use of the invention.

In particular, the recombinant bacterium may be selected from the group consisting of bacteria belonging to the genera *Allobaculum* (e.g. *Allobaculum stercoricanis), Akkermansia* (e.g. *Akkermansia muciniphila), Anaerostipes* (e.g. *Anaerostipes hadrus, Anaerostipes caccae* and *Anaerostipes butyraticus), Bifidobacterium, Bacillus* (e.g. *Bacillus subtilis* and *Bacillus clausii), Propionibacterium, Bacteroides, Eubacterium, Enterococcus, Ruminococcus* (e.g. *Ruminococcus gnavus), Roseburia* (e.g. *Roseburia hominis)* and *Faecalibacterium* (e.g. *Faecalibacterium prausnitzii), Escherichia coli,* and lactic acid bacteria, in particular lactic acid bacteria belonging to the genera *Lactobacillus* (e.g. *Lactobacillus casei, Lactobacillus reuteri, Lactobacillus plantarum, Lactobacillus taiwanensis, Lactobacillus johnsonii, Lactobacillus animalis, Lactobacillus murinus, Lactobacillus salivarius, Lactobacillus gasseri, Lactobacillus bulgaricus,* and *Lactobacillus delbrueckii subsp. bulgaricus), Lactococcus* (e.g. *Lactococcus lactis*) and *Streptococcus* (e.g. *Streptococcus thermophilus).* Preferably, the recombinant bacterium is selected from the group consisting of bacteria belonging to the genera *Lactobacillus* and *Lactococcus,* in particular from the group consisting of *Lactobacillus casei* and *Lactococcus lactis.*

In some other embodiments, the composition used in the present invention comprises xanthurenic acid, a derivative thereof or any pharmaceutically acceptable salt or solvate thereof, wherein the xanthurenic acid derivative is of formula (I) 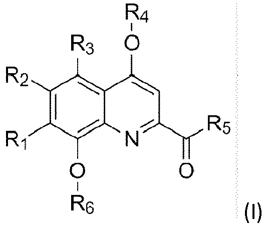 wherein
R₁, R₂ and R₃ are independently selected from the group consisting of a hydrogen atom, an oxygen atom, a halogen atom, a C₁₋₁₀ alkyl, C₂₋₁₀ alkenyl or C₂₋₁₀ alkynyl group optionally substituted by a halogen atom;
R₄ and R₆ are independently selected from the group consisting of a hydrogen atom and a C₁₋₁₀ alkyl group, or R₄ and/or R₆ are absent; and
R₅ is selected from the group consisting of a hydroxyl group, NHR₇, NR₇R₇ and a C₁₋₁₀ alkyl or alkoxy group, wherein R₇ is selected from the group consisting of a hydrogen atom and a C₁₋₁₀ alkyl group.

In a particular embodiment,
R₁, R₂ and R₃ are independently selected from the group consisting of a hydrogen atom, an oxygen atom a halogen atom, a C₁₋₆ alkyl, C₂₋₆ alkenyl or C₂₋₆ alkynyl group optionally substituted by a halogen atom;
R₄ and R₆ are independently selected from the group consisting of a hydrogen atom and a C₁₋₆ alkyl group, or R₄ and/or R₆ are absent; and
R₅ is selected from the group consisting of a hydroxyl group, NHR₇, NR₇R₇ and a C₁₋₆ alkyl or alkoxy group, wherein R₇ is selected from the group consisting of a hydrogen atom and a C₁₋₆ alkyl group.

As used herein, the term "alkyl" refers to a univalent radical containing only carbon and hydrogen atoms arranged in a linear or branched chain. (C₁-C₃)-alkyl groups include methyl, ethyl, propyl, or isopropyl. Preferably, the (C₁-C₃)-alkyl group is methyl of ethyl, more preferably methyl. (C₁-C₆)-alkyl groups include methyl, ethyl, propyl, isopropyl, butyl, isobutyl, tert-butyl, pentyl or hexyl.

As used herein, the term "alkenyl" refers to an unsaturated, linear or branched aliphatic group comprising at least one carbon-carbon double bound. The term "(C₂-C₆)alkenyl" more specifically means ethenyl, propenyl, isopropenyl, butenyl, isobutenyl, pentenyl, or hexenyl.

The term "alkynyl" refers to an unsaturated, linear or branched aliphatic group comprising at least one carbon-carbon triple bound. The term "(C₂-C₆)alkynyl" more specifically means ethynyl, propynyl, butynyl, pentynyl, isopentynyl, or hexynyl.

The term "halogen" corresponds to a fluorine, chlorine, bromine, or iodine atom.

Example of xanthurenic acid derivatives include, but are not limited to, oxo-xanthurenic acid (OXA) and di-oxo-xanthurenic acid (DOXA).

| | |
|---|---|
| 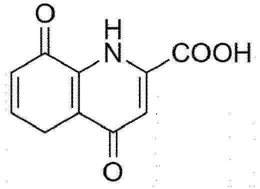 | Oxo-xanthurenic acid (OXA) |
| 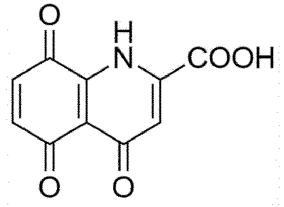 | Di-oxo-xanthurenic acid (DOXA) |

In a preferred embodiment, the composition comprises xanthurenic acid or any pharmaceutically acceptable salt or solvate thereof.

### Xanthurenic acid is of formula (II)

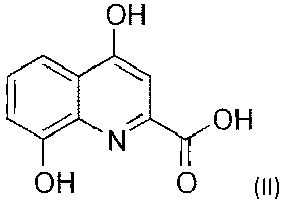

It will be apparent to one skilled in the art that certain compounds described in the present disclosure may exist in tautomeric forms, all such tautomeric forms of the compounds being within the scope of the disclosure. In particular, the term "xanthurenic acid" includes the compound of formula (II) and its tautomeric forms such as compounds of formulas (III) and (IV)

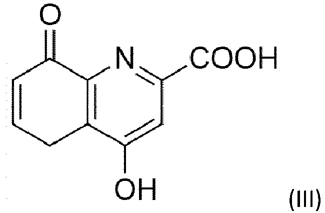

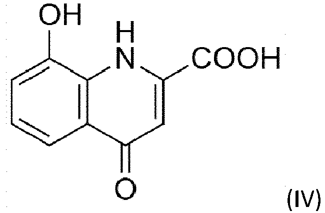

Pharmaceutically acceptable salts of xanthurenic acid or a derivative thereof are salts which are non-toxic for a patient and suitable for maintaining the stability of the compound and allowing the delivery of said compound to target cells or tissue. Pharmaceutically acceptable salts are well known in the art.

As used herein, the term "solvate" refers to a solvent addition form that contains either stoichiometric or non stoichiometric amounts of solvent. Some compounds have a tendency to trap a fixed molar ratio of solvent molecules in the crystalline solid state, thus forming a solvate. If the solvent is water, the solvate formed is a hydrate. When the solvent is alcohol, the solvate formed is an alcoholate. Hydrates are formed by the combination of one or more molecules of water with one of the substances in which the water retains its molecular state as H₂O, such combination being able to form one or more hydrates.

The composition used in the invention may be a pharmaceutical composition, a food composition or food supplement.

In embodiments wherein the composition is a pharmaceutical composition, the active compound as defined above, i.e. a kynurenine aminotransferase, a live recombinant bacterium which has been genetically modified to express and secrete said kynurenine aminotransferase, and/or xanthurenic acid or a derivative thereof, or any pharmaceutically acceptable salt or solvate thereof, may be combined with pharmaceutically acceptable excipients, and optionally sustained-release matrices, such as biodegradable polymers, to form therapeutic compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutically acceptable excipients that can be used in the composition according to the invention are well known to the skilled person and may vary according to the disease to be treated and the administration route.

The composition used in the invention can be administered by any method suitable for depositing in the gastrointestinal tract, preferably the small intestine and/or the colon, of the subject to be treated. In particular, the composition can be administered by enteral or parenteral route, preferably by oral, sublingual, subcutaneous, intramuscular, intravenous, transdermal, local or rectal administration route. Preferably, the composition is administered, or is adapted to be administered, by rectal or oral route.

In particular, in some embodiments wherein the composition comprises a kynurenine aminotransferase and/or xanthurenic acid or a derivative thereof, said composition is preferably administered by oral, rectal, subcutaneous or intravenous route. In some other embodiments wherein the composition comprises a live recombinant bacterium, said composition is preferably administered by oral or rectal route.

In an embodiment, the pharmaceutical composition is to be administered by oral route. For oral administration, the pharmaceutical composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants may also be necessary in the tablets to facilitate break-up of the tablet. Disintegrants include starches, clays, celluloses, algins, gums and crosslinked polymers. Moreover, lubricants and glidants may also be included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Well-known thickening agents may also be added to compositions such as corn starch, agar, natural or synthetic gums, resins, methylcellulose, sodium carboxymethylcellulose, guar, xanthan and the like. Preservatives may also be included in the composition, including methylparaben, propylparaben, benzyl alcohol and ethylene diamine tetraacetate salts.

Preferably, for oral administration, the composition is in a gastro-resistant oral form allowing the active compounds contained in the composition, to pass the stomach and be released into the intestine. The material that can be used in enteric coatings includes, for example, alginic acid, cellulose acetate phthalate, plastics, waxes, shellac and fatty acids (e.g. stearic acid or palmitic acid).

In another embodiment, the pharmaceutical composition is to be administrated by rectal route. Suitable rectal-route forms include, but are not limited to, suppository and enema. In particular, the active compounds can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Compositions used in the invention may be formulated to release the active compounds substantially immediately upon administration or at any predetermined time or time period after administration.

The pharmaceutical composition used in the present invention may be used in combination with, or may further comprise, at least one additional active ingredient, in particular at least one bacterial probiotic and/or at least one prebiotic and/or at least one drug useful in the treatment of inflammatory bowel disease.

The term "bacterial probiotic" has its general meaning in the art and refers to a useful bacterium that can bring a beneficial action to the host health, i.e. which is applicable to the prevention, treatment or cure of a disease or condition of the host, preferably a human being. This term may refer to a dead or living bacterium. Preferably, this term refers to a living bacterium (also named live biotherapeutic product). Preferably, said bacterial probiotic exhibits anti-inflammatory activity. Such bacterial probiotics can be selected, for example, from the group consisting of bacteria belonging to the genera *Allobaculum* (e.g. *Allobaculum stercoricanis), Akkermansia* (e.g. *Akkermansia muciniphila), Anaerostipes* (e.g. *Anaerostipes hadrus, Anaerostipes caccae* and *Anaerostipes butyraticus), Bifidobacterium, Bacillus* (e.g. *Bacillus subtilis* and *Bacillus clausii), Propionibacterium, Bacteroides, Eubacterium, Enterococcus, Ruminococcus* (e.g. *Ruminococcus gnavus), Roseburia* (e.g. *Roseburia hominis)* and *Faecalibacterium* (e.g. *Faecalibacterium prausnitzii), Escherichia coli,* and lactic acid bacteria, in particular lactic acid bacteria belonging to the genera *Lactobacillus* (e.g. *Lactobacillus reuteri, Lactobacillus plantarum, Lactobacillus taiwanensis, Lactobacillus johnsonii, Lactobacillus animalis, Lactobacillus murinus, Lactobacillus salivarius, Lactobacillus gasseri, Lactobacillus bulgaricus,* and *Lactobacillus delbrueckii subsp. Bulgaricus), Lactococcus* and *Streptococcus* (e.g. *Streptococcus thermophilus).*

As used here, a "prebiotic" refers to an ingredient that can induce specific changes in both the composition and/or activity of the administered probiotic(s) and/or gastrointestinal microbiome that may confer benefits to the host. Preferably, the prebiotic can be degraded by the probiotic(s) and may increase the shelf life of said probiotic(s) after administration to the patient. Examples of prebiotics include, but are not limited to, complex carbohydrates, polyphenols, amino acids, peptides, minerals, or other nutritional components essential for the survival of the probiotic(s). In particular, the prebiotic may selected from the group consisting of inulin, inositol, tagatose, lactulose, alpha-glucan oligosaccharide, trans-galacto-oligosaccharides (TOS), fructo-oligosaccharides (FOS), galacto-oligosaccharides (GOS), xylooligosaccharides (XOS) and a mixture thereof.

Examples of drugs useful in the treatment of inflammatory bowel diseases include, but are not limited to, corticosteroids, 5-aminosalicylates, immunosuppressors such as cyclosporin, azathioprine, 6-mercaptopurine, methotrexate, anti-TNF agents (such as infliximab, adalimumab, golimumab, certolizumab), anti-integrin agents (such as natalizumab, vedolizumab) anti-IL12 and/or -IL23 antibodies (such as ustekinumab), JAK inhibitors (such as tofacitinib), antibiotics, anti-diarrheals, pain relievers, iron supplements, vitamin B-12, calcium and vitamin D.

In others embodiments, the composition may be a food composition or a food supplement.

By "food composition" is meant any composition comprising food ingredients such as macronutrients, micronutrients, vitamins and/or minerals. The food composition may be intended for human or animal consumption and may be a liquid, paste or solid. Examples of food compositions include, but are not limited to dairy products such as cheese, butter, cream, yoghurt, fermented milk, ice cream, cooked products such as bread, biscuits and cakes, fruit products such as fruit juice, fruit compote or fruit paste, soy food products, starch-based food products, edible oil compositions, spreads, breakfast cereals, infant formula, food bars (e.g. cereal bars, breakfast bars, energy bars, nutrition bars), chewing gum, beverages, drinking supplements (powders to be added to a beverage).

As used herein, the term "food supplement" refers to any composition which is formulated and administered separately from other foods to complement the nutritional intakes of a subject, i.e. a human or an animal. This supplement may be in any suitable form well known to those skilled in the art, preferably in the form of dietetic food or oral supplementation.

In particular embodiments, the pharmaceutical composition, food composition or food supplement further comprises nicotinamide adenine dinucleotide (NAD), or a precursor thereof, or is to be used in combination with NAD, or a precursor thereof. NAD may be administered in oxidized (NAD+) or reduced (NADH) form. Examples of NAD precursors include, but are not limited to, nicotinamide (NAM), nicotinic acid (NA), nicotinamide mononucleotide (NMN), and nicotinamide riboside (NR).

Preferably, in these embodiments, the pharmaceutical composition, food composition or food supplement comprises a KAT enzyme.

Preferably, the nicotinamide adenine dinucleotide or its precursor is administered by oral or intravenous route. Depending on the nature and the route of administration of the composition, the nicotinamide adenine dinucleotide or its precursor and the composition may be administered simultaneously or separately, via the same route or via a different route.

The composition as described above is used in the treatment of an inflammatory bowel disease.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of the disease or symptoms associated with it. In other embodiments, this term refers to minimizing the spread or worsening of the disease resulting from the administration of one or more therapeutic agents, e.g. the composition of the invention, to a subject with such a disease.

As used herein, "inflammatory bowel diseases" or "IBD" refers to any of a variety of diseases characterized by inflammation of all or part of the intestines. Examples of inflammatory bowel diseases include, but are not limited to, Crohn's disease (such as enteritis, ileitis, colitis, ileocolitis, gastroduodenal and perianal Crohn's), ulcerative colitis (such as ulcerative enterocolitis, ulcerative ileocolitis, ulcerative proctitis, ulcerative rectosigmoiditis, pseudopolyposis of colon, mucosal proctocolitis, and left-sided or extensive ulcerative colitis), indeterminate colitis (IC), other noninfective gastroenteritis, enteritis, enterocolitis and colitis (such as non-microscopic colitis including collagenous colitis and lymphocytic colitis, radiation-induced, toxic-induced, allergic-induced, dietetic-induced, ischemic, eosinophilic gastroenteritis, enteritis, enterocolitis or colitis, segmental colitis associated with diverticula, diversion colitis and Behcet's colitis) as well as pouchitis.

Preferably, the inflammatory bowel disease is selected from the group consisting of Crohn's disease and ulcerative colitis.

The composition of the invention may be used in combination with an anti-inflammatory diet or a low carb diet. In particular, such anti-inflammatory diet may comprise 1) avoiding pro-inflammatory food, in particular such as refined carbohydrates, fried foods, soft drinks and other sweetened beverages, red meat and processed meat; 2) favoring the intake of anti-inflammatory food, such as olive oil, green leafy vegetables like spinach or kale, nuts like almonds and walnuts, fatty fish like salmon, mackerel, tuna and sardines, fruits like strawberries, blueberries, cherries, oranges, apples and tomatoes.

The subject to be treated with the composition of the invention is an animal, preferably a mammal. In an embodiment, the subject is a domestic or farmed animal such as dogs, cats, cows, sheep, horses or rodents. In a preferred embodiment, the subject is a human, including adult, child, newborns and human at the prenatal stage. As used herein, the terms "subject", "individual" and "patient" are interchangeable.

The dosage of the active compound(s) as defined above, i.e. a kynurenine aminotransferase, a live recombinant bacterium which has been genetically modified to express and secrete said kynurenine aminotransferase, and/or xanthurenic acid or a derivative thereof, or any pharmaceutically acceptable salt or solvate thereof, may be appropriately adjusted according to criteria such as age, symptoms, body weight, and intended application such as to obtain a therapeutically efficient amount. The term "therapeutically efficient amount" as employed herein refers to the amount necessary for having a beneficial impact on the disease to be treated, i.e. to prevent, remove or reduce at least one deleterious effect of the disease. A therapeutically efficient amount is preferably defined as the amount necessary for having an impact on intestinal inflammation or any symptom of the disease such as diarrhea, fever or pain.

In some embodiments, the composition may comprise from 10³ to 10¹¹ live recombinant bacterium cells as defined above, per mg of composition. In particular, the amount of live recombinant bacterium cells as defined above, ingested per day may be from 1×10⁶ to 1×10¹¹ CFU/body, preferably 0.1×10⁹ to 10×10⁹ CFU/body, and more preferably 0.3×10⁹ to 5×10⁹ cells/body. The content of live recombinant bacterium cells as defined above, contained in an orally ingested composition of the invention may be for example from 1% to 100% (w/w, i.e. bacteria dry weight/total dry weight of the composition), preferably from 1% to 75% (w/w), and more preferably from 5% to 50% (w/w).

In some other embodiments, the composition may comprise from 0.001 mg to 1 mg of KAT enzyme(s) as defined above, per mg of composition. In particular, the amount of KAT enzyme(s) as defined above, ingested per day may be from 0.001 mg /body to 100 mg / body. The content of KAT enzyme(s) as defined above, contained in an orally ingested composition of the invention may be for example from 0.1% to 100% (w/w, i.e. KAT enzyme weight/total dry weight of the composition), preferably from 0.1% to 75% (w/w).

In some other embodiments, the composition may comprise from 0.001 mg to 1 mg of xanthurenic acid or a derivative thereof as defined above, per mg of composition. In particular, the amount of xanthurenic acid or a derivative thereof as defined above, ingested per day may be from 0.001 mg /body to 100 mg / body. The content of xanthurenic acid or a derivative thereof as defined above, contained in an orally ingested composition of the invention may be for example from 0.1% to 100% (w/w, i.e. xanthurenic acid or derivative weight/total dry weight of the composition), preferably from 0.1% to 75% (w/w), and more preferably from 0.5% to 50% (w/w).

The composition of the invention may be administered as a single dose or in multiple doses. In particular, depending on the subject's age or physiological condition, daily doses may be divided to facilitate administration, for example with one administration in the morning and another in the evening.

In some embodiments, the composition is to be administered regularly, preferably between every day and every month, more preferably between every day and every two weeks, more preferably between every day and every week. In some particular embodiments, the composition is to be administered every day.

The duration of treatment with the composition of the invention may be comprised between 1 day and several years, preferably between 1 day and one year, more preferably between 1 day and 6 months.

All the references cited in this description are incorporated by reference in the present application. Others features and advantages of the invention will become clearer in the following examples which are given for purposes of illustration and not by way of limitation.

### EXAMPLES

### Example 1

### Materials and methods

### Mice

Male and female C57BL/6JRj mice were purchased from Janvier (France). *AhR*^{-/-} mice on the C57BL/6JRj background were obtained from the Jackson laboratory (JAX stock #002831) (Fernandez-Salguero et al. Science. 1995, 268, 722-726) and were bred at Saint-Antoine Research Center. *Il22*^{-/-} mice (Kreymborg et al., J. Immunol. 2007, 179, 8098-8104) were obtained and bred at the Transgenose Institute (TAMM-CNRS, Orleans, France). All the mice were housed at the IERP facility (INRA, Jouy-en-Josas, France), which is accredited by the French "Direction Départementale de la Protection des Populations (DDPP78)". All experiments were performed in accordance with the Comite d'Ethique en Experimentation Animale (COMETHEA C2EA - 45, Jouy en Josas, France).

### DSS model

C57BL/6JRj from Janvier lab, 7 weeks old, were housed in our specific pathogen free animal facility at IERP (INRA, Jouy en Josas, France). Five to ten female mice per group were used in all experiment, were maintained in a temperature controlled (23°C) facility with a strict 12h light/dark cycles and were given free access to food and water. After one acclimation week, we changed water with DSS 2% (MP Biomedical) during 7 days (day 0 to day 7), and a recovery period was practiced until day 12 (during 5 days). Body weight loss and DAI (disease activity index) were performed daily. After 12 days the samples were harvested.

| **Body weight loss** | | | | |
|---|---|---|---|---|
| 0 | | No loss | | |
| 1 | | 1-5% loss of body weight | | |
| 2 | | 6-10% loss of body weight | | |
| 3 | | 10-20% loss of body weight | | |
| 4 | | >20% loss of body weight | | |
| | | | | |

| **Stool consitency** | | | | |
|---|---|---|---|---|
| 0 | | Normal Feces | | |
| 1 | | Loose stool | | |
| 2 | | Watery diarrhea | | |
| 3 | | Slimy diarrhea, little blood | | |
| 4 | | Severe watery diarrhea with blood | | |
| | | | | |

| **Blood in stool** | | | | |
|---|---|---|---|---|
| 0 | | No blood | | |
| 1 | | Presence of blood | | |
| 2 | | Visible blood - low quantity | | |
| 3 | | Visible blood - medium quantity | | |
| 4 | | Visible blood - high quantity | | |

### Colon histology

Colon distal part (cut in three parts) was harvested at day 12 after DSS model and put directly in PFA (Rothi^{®}- Histofix 4%) during 48h and transferred in ethanol 70% until the standard pre-fixation automat and paraffin embedded. HES coloration was practiced on 5µm cut. Scoring was performed on the worst cut and scored as previously described (Sokol et al., Gastroenterology. 2013 Sep;145(3):591-601.e3.).

### Statistical analysis

Data were analyzed using Prism version 7 (Graphpad Software, San Diego, USA). The non-parametric Mann Withney test or the parametric one-way ANOVA tests with multiple Bonferroni's comparison tests were performed. Values are expressed as mean ± SEM. Statistical significance was defined at a p-value **** <0.0001, *** <0.001, ** <0.01, * <0.05.

### Results

The kynurenine pathway was monitored as previously described (Lefèvre et al., Talanta 195 (2019) 593-598) during a model of intestinal inflammation induced by DSS (Dextran Sodium Sulfate) in mice. The metabolites composing this pathway are tryptophan, kynurenine (KYNU), kynurenic acid (KYNA), 3-Hydroxykynurenine (3HK), xanthurenic acid (XANA), 3-Hydroxyanthranilic acid (3-HAA), picolinic acid and quinolinic acid. It was observed that the dynamics of these metabolites differed from one to the other. In particular, the dynamics of kynurenine and quinolinic acid correlate with inflammation, while that of xanthurenic acid or kynurenic acid had the opposite dynamic. This phenomenon is particularly apparent at the peak of inflammation on D9 (Figure 1A-B).

This phenomenon was then investigated in a human context. For this purpose, fecal and serum samples from patients enrolled in a clinical trial evaluating fecal microbiota transplantation in Crohn's disease (NCT02097797) were analyzed. Metabolomic analysis targeting tryptophan metabolites was performed on these samples and correlations were made with various clinical, endoscopic and biological markers of the disease activity. A negative correlation was observed between several markers of disease activity with KYNA and XANA (as well as the ratio between KYNA, XANA and their precursors, kynurenine and 3-hydroxykynurenine, respectively). The opposite was observed with kynurenine and quinolinic acid (data not shown). These results, therefore, confirmed the human relevance of the data identified in mice.

Based on these results, it was hypothesized that XANA and KYNA might have anti-inflammatory effects. To further explore the effect of these metabolites, XANA and KYNA were administered to mice submitted to DSS-induced colitis. Each day, 8mg of KYNA and 6mg of XANA were delivered orally to each mouse. Doses are in agreement with literature for KYNA (R.Gill and G.N. Woodruff, 1990, European Journal of Pharmacology). The weight loss and the activity index of colitis were measured daily and showed that these two metabolites have an anti-inflammatory effect (Fig. 2A-B). At the final time point of the model, on day twelve, the colon length in these mice is longer, and the histological score lower (Fig. 2C-E), confirming the protective effect of KYNA and XANA. The colonic expression of many pro-inflammatory cytokines such as IL-1 or IL-17, (measured by nanostring^{®} technology) was also reduced in mice treated with XANA or KYNA (Data not shown). In addition, intraperitoneal injection of XANA and KYNA also induces protection similar to that observed when these metabolites are given orally, suggesting a systemic effect (Fig. 3A-E).

### Example 2

### Materials and methods

### Cloning of murine Alpha-AminoaDipate AminoTransferase (AADAT) in Escherichia coli.

The codon usage of gene encoding murine AADAT (muAADAT) and synthesized by the company GeneArt (pMA:muAADAT plasmid). The DNA fragment coding for muAADAT was then recovered after digestion with *Nhe*l and *Xho*l restriction enzymes and cloned into pStaby 1 vector (DelphiGenetics) previously digested with the same enzymes. The use of pStaby 1 plasmid (Fig.4A) allows the introduction of a C-terminal six-Histidine tag (His-tag), allowing subsequent purification of muAADAT using affinity chromatography and under the control of the phage T7 RNA polymerase promoter (T7 polymerase). The final vector pStaby:muAADAT (Fig.4B) was transferred into T7 Express Competent *E. coli* (NEB) and transformants were grown at 37°C overnight (ON) in 10 mL of Luria-Bertani medium containing ampicillin (Amp, 100 µg/mlL) with shaking at 180 rpm. Plasmid DNA was extracted from positive clones and sequenced to confirm their identity.

### Expression and purification of the recombinant murine AADAT protein in E. coli.

*E. coli* strain expressing muAADAT was cultured at 37°C ON in 100 mL of LB supplemented with Amp 100 µg/ml and with shaking at 180 rpm and then cultured in 10 L of LB Amp 100 µg/ml at 37°C. When an optical density (OD 600 nm) of 0.8-1.0 was reached, gene expression was induced by the addition of 0.25 mM IPTG and the cultures incubated at 16°C ON with stirring at 180 rpm (Fig.4C). Bacteria were harvested by centrifugation and the cell pellets washed with PBS and resuspended in 100 ml of Binding Buffer (PBS buffer pH 7.4-300 mM NaCl supplemented with 0.1% 10X triton and 1X protease inhibitors. Rock). The cells were then sonicated in ice with an amplitude of 40%, 5 s then 3 s of rest for 20 min. The lysate is then centrifuged at 15,000 g for 30 min at 4°C to separate the soluble fraction from the cell pellet. The soluble fraction containing AADAT was purified by affinity chromatography. The 100 mL of supernatant were incubated with 4 mL of Ni-NTA agarose resin (ref. R901-15 from Invitrogen) at 4°C for 1 h then deposited in a BioRad column. The AADAT protein fixed to the resin was washed with 100 mL of PBS buffer pH 7.4-500 mM NaCl, then 25 mL of PBS buffer pH 7.4-300 mM NaCl-20 mM imidazole and 10 mL of buffer. PBS buffer pH 7.4-300 mM NaCl-40 mM imidazole. The AADAT protein was eluted with 8 ml of PBS buffer pH 7.4-300 mM NaCl-300 mM imidazole (Fig. 4D). The fractions 2-6 were pooled and dialyzed against 1 L of PBS, 50% glycerol-300 mM NaCl Specta/Port 6. The protein was stored at -20°C. A pre-cast BioRad mini-protean TGX stain free, 4-20% gel was carried out to control the different stages of the purification.

### AADAT activity test verification.

The activity test is based on the disappearance of kynurenine and 3-hydroxykynurenin which are substrates of AADAT. A reaction mixture (50 µL final volume) containing 10 mM L-kynurenine or 3-hydroxykynurenin, 2 mM α-oxoglutarate, 40 µM PLP (pyridoxal 5'-phosphate) and 0 or 1 µL of the purified protein sample prepared in buffer 100 mM potassium phosphate (pH 7.4). Then, the mixture was incubated at 37 ° C for 15 min, and the reaction was stopped by the addition of an equal volume of 30% acetic acid. The supernatant of the reaction mixture, obtained by centrifugation at 3000 g for 10 min at 4 °C, was mixed equally with Ehrlich's solution and incubated 15 min at room temperature to have a colorimetric reaction. In parallel, a standard range of kynurenine or 3-hydroxykynurenin from 0 µM to 1000 µM was made under the same conditions. The amount of kynurenine or 3-hydroxykynurenin present in the sample was measured with a spectrophotometer at an OD of 492 nm and calculated using the standard range (Fig.4E). A verification of the production of KYNA and XANA *in vitro* via the action of our enzyme during 15min in the presence of KYNU (1000 µM) was carried out (Fig. 4F). A dose effect can be observed from the transformation of KYNU into KYNA and XANA.

### Results

Once all the checks had been made, the purified AADAT protein was then administered intraperitoneally to mice, during the model of colitis induced by DSS. Taking into account the degradation of the KYNU and 3HK of our enzyme (Fig. 4E) and the production of KYNA and XANA in the supernatant (Fig. 4F), with 1µL, three doses were evaluated (10, 1 and 0.1µL per day and per mouse). The results obtained show protection, with a dose effect, both in terms of weight loss and clinical score, but also colon length on day 12 or histological score (Fig. 5A-E). The protective effect is also seen with the two lower doses.

## Claims

1. A composition for use in the treatment of an inflammatory bowel disease, wherein said composition comprises
- a kynurenine aminotransferase (KAT),
- a living recombinant bacterium which has been genetically modified to express and secrete said kynurenine aminotransferase, and/or
- a product of said kynurenine aminotransferase which is xanthurenic acid, a derivative thereof, or any pharmaceutically acceptable salt or solvate thereof.

2. The composition for use according to claim 1, wherein the inflammatory bowel disease is selected from the group consisting of Crohn's disease, ulcerative colitis, indeterminate colitis (IC), other noninfective gastroenteritis, enteritis, enterocolitis and colitis, and pouchitis.

3. The composition for use according to claim 1 or 2, wherein the inflammatory bowel disease is selected from Crohn's disease and ulcerative colitis.

4. The composition for use according to any of claims 1 to 3, wherein the kynurenine aminotransferase is selected from the group consisting of human kynurenine--oxoglutarate transaminase 1 (KAT I), human kynurenine/alpha-aminoadipate aminotransferase (KAT II), human kynurenine--oxoglutarate transaminase 3 (KAT III), human mitochondrial aspartate aminotransferase (KAT IV), orthologs thereof, and variants thereof, said variants having at least 80% sequence identity to human KAT I, human KAT II, human KAT III, human KAT IV or to any ortholog thereof, and exhibiting kynurenine aminotransferase activity.

5. The composition for use according to any of claims 1 to 4, wherein the kynurenine aminotransferase is selected from the group consisting of human KAT II, human KAT III, human KAT IV, orthologs thereof, and variants thereof, said variants having at least 80% sequence identity to human KAT II, human KAT III, human KAT IV or to any ortholog thereof, and exhibiting kynurenine aminotransferase activity.

6. The composition for use according to any of claims 1 to 5, wherein the kynurenine aminotransferase is selected from the group consisting of human KAT II, orthologs thereof, and variants thereof, said variants having at least 80% sequence identity to human KAT II or to any ortholog thereof, and exhibiting kynurenine aminotransferase activity.

7. The composition for use according to any of claims 1 to 4, wherein the kynurenine aminotransferase is selected from the group consisting of KAT proteins of SEQ ID NO: 1 to 32, and variants thereof having at least 80% sequence identity to any sequence of SEQ ID NO: 1 to 32 and exhibiting kynurenine aminotransferase activity.

8. The composition for use according to any of claims 1 to 7, wherein the kynurenine aminotransferase is selected from the group consisting of KAT proteins of SEQ ID NO: 10 to 16, and variants thereof having at least 80% sequence identity to any sequence of SEQ ID NO: 10 to 16 and exhibiting kynurenine aminotransferase activity.

9. The composition for use according to any of claims 1 to 8, wherein said composition comprises said kynurenine aminotransferase.

10. The composition for use according to any of claims 1 to 9, wherein said composition comprises a recombinant bacterium which has been genetically modified to express and secrete said kynurenine aminotransferase.

11. The composition for use according to any of claims 1 to 10, wherein said recombinant bacterium is selected from the group consisting of bacteria belonging to the genera *Allobaculum, Adlercreutzia, Anaerostipes, Bifidobacterium, Propionibacterium, Bacteroides, Eubacterium, Enterococcus, Ruminococcus* and *Faecalibacterium, Escherichia coli,* and lactic acid bacteria such as bacteria belonging to the genera *Lactobacillus, Lactococcus* and *Streptococcus.*

12. The composition for use according to any of claims 1 to 11, wherein said composition comprises xanthurenic acid, a derivative thereof or any pharmaceutically acceptable salt or solvate thereof, wherein the xanthurenic acid derivative is of formula (I) 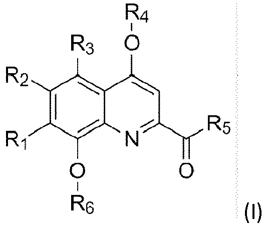 wherein
R₁, R₂ and R₃ are independently selected from the group consisting of a hydrogen atom, an oxygen atom, a halogen atom, a C₁₋₁₀ alkyl, C₂₋₁₀ alkenyl or C₂₋₁₀ alkynyl group optionally substituted by a halogen atom;
R₄ and R₆ are independently selected from the group consisting of a hydrogen atom and a C₁₋₁₀ alkyl group, or R₄ and/or R₆ are absent; and
R₅ is selected from the group consisting of a hydroxyl group, NHR₇, NR₇R₇ and a C₁₋₁₀ alkyl or alkoxy group, wherein R₇ is selected from the group consisting of a hydrogen atom and a C₁₋₁₀ alkyl group.

13. The composition for use according to any of claims 1 to 12, wherein the xanthurenic acid derivative is selected from the group consisting of oxo-xanthurenic acid (OXA) and di-oxo-xanthurenic acid (DOXA).

14. The composition for use according to any of claims 1 to 13, wherein said composition comprises xanthurenic acid or any pharmaceutically acceptable salt or solvate thereof.

15. The composition for use according to any of claims 1 to 14, wherein said composition further comprises nicotinamide adenine dinucleotide or a precursor thereof, or is to be used in combination with nicotinamide adenine dinucleotide or a precursor thereof.
